# EUROPEAN PATENT SPECIFICATION

(11) **EP 2 601 847 B1**
(45) Date of publication and mention of the grant of the patent: **26.08.2015**
(21) Application number: 12193029.1
(22) Date of filing: 16.11.2012
(51) Int. Cl.: A23L 1/29, A23L 1/30, A61K 31/445, A61K 31/57, A61P 3/10, A61P 3/04

(54) **A nutraceutical compound for treating excess of weight**
Ein Nutrazeutikum zur Behandlung von Übergewicht
Un alicament pour le traitement d'excès de poids

(30) Priority: 17.11.2011 IT MI20112086
(43) Date of publication of application: 12.06.2013
(73) Proprietor: ROTTAPHARM S.P.A., 20122 Milano (IT)
(72) Inventor: Sirtori, Cesare, 20122 MILANO (Mi) (IT)
(74) Representative: Lunati & Mazzoni S.r.L.

(56) References cited:
- US-A1- 2007 009 615
- JING-JIE TANG ET AL: "Inhibition of SREBP by a Small Molecule, Betulin, Improves Hyperlipidemia and Insulin Resistance and Reduces Atherosclerotic Plaques", CELL METABOLISM, CELL PRESS, UNITED STATES, vol. 13, no. 1, 4 November 2010 (2010-11-04), pages 44-56, XP028362361, ISSN: 1550-4131, DOI: 10.1016/J.CMET.2010.12.004 [retrieved on 2010-12-06]
- DATABASE WPI Week 201202 Thomson Scientific, London, GB; AN 2011-N81912 XP002678631, & CN 102 204 671 A (NEST INC) 5 October 2011 (2011-10-05)
- DATABASE WPI Week 200957 Thomson Scientific, London, GB; AN 2009-M79735 XP002678632, & WO 2009/101698 A1 (PROJECT M CO LTD) 20 August 2009 (2009-08-20)

## Description

The present invention relates to a nutraceutical compound for treating excess of weight of the type as recited in the preamble of Claim 1.

In particular, the invention concerns a nutraceutical compound able to reduce body weight and which can thus be used as a treatment to accompany a low calorie diet.

As is known, according to the World Health Organisation (WHO) overweight and, especially, obesity represent one of the biggest public health problems in the world. In recent years there has been a global epidemic, which is taking over many parts of the world. If immediate action is not taken it will cause serious health disorders in the coming years.

Obesity and overweight are associated with premature death and are now universally recognised as risk factors for the main chronic diseases: cardiovascular disease, stroke, diabetes, certain forms of cancer (of the endometrium, colon and rectum, kidney, gallbladder and post-menopause breast cancer), gallbladder disorders, osteoarthritis.

Another important problem, of increasing importance not only in industrialised countries, is the high risk of diabetes among people who are overweight.

A further serious problem of increasing importance, is the incidence of obesity among children and adolescents, who are exposed, from a very early age, to the risk of developing respiratory illnesses, joint and movement problems, but also psychological disorders and problems with digestion.

Moreover, children who are overweight tend to grow into overweight adults, and are thus more likely to develop risk factors for cardiovascular disease (hypertension, coronary disease, risk of heart attack) and metabolic disorders while still young.

In view of the problems mentioned above and the importance of maintaining a correct body weight, more and more medicines, supplements and other similar elements that permit people to lose weight are being developed.

At present, one of the most effective methods for helping people lose weight envisages the use of drugs known as "slimming tablets" made of caffeine, ephedrine or amphetamine which raise the rate of metabolism and thus increase the calories burned.

While having important advantages, these drugs also have some no less important contraindications.

The use of amphetamines can lead to numerous problems such as, for example, dehydration, gastrointestinal disorders, headache, hypertension, arrhythmias, angina pectoris, infarction, cardiac valve disease, pulmonary hypertension, increased risk of stroke.

Another problem, related to the use of said pills, consists of the fact that the body gradually builds up a sort of resistance to the slimming effects of the dug making it necessary to increase the dose and consequently the risks associated with the use of amphetamines. The use of amphetamines and similar substances leads to dependence and addiction which, when the treatment is stopped, cause mental depression and fatigue.

For these reasons, other drugs have been developed which exploit other active ingredients that can help people to lose weight.

Examples of said drugs are described in US 2007009615, by Jing-Jie Tang in "Inhibition of SREBP by a Small Molecule, Betulin, Improves Hyperlipidemia and Insulin Resistance and Reduces Atherosclerotic Plaques" and in Database WPI (see Week 201202, Thomson Scientific, London; AN 2011-N81912 XP002678631 & CN 102204671 A; and Week 200957, Thomson Scientific, London; AN 2009-M79735 XP002678632 & WO 2009101698 A1).

One of the most important drugs is Sibutramine^{®} which acts on the central nervous system exploiting a mechanism of action similar to that of some antidepressant drugs. In particular, it inhibits the synaptic reuptake of norepinephrine, serotonin and, to a lesser extent, dopamine and thus promotes a natural feeling of satiety after meals.

While producing positive results, Sibutramine^{®} also has some important side effects as a result of which it has been withdrawn from the market.

Since Sibutramine^{®} acts on the nervous system, it can give rise to headache, insomnia, anorexia. Another problem is that it causes an increase in arterial pressure and heart rate and is therefore contraindicated for anyone with hypertension and a history of coronary disease, congestive heart failure, tachycardia, peripheral arterial occlusive disease, arrhythmia or cerebrovascular disease (stroke or TIA). Due to this series of important clinical problems, the drug was recently taken off the market.

Another type of drug, such as Orlistat^{®}, for example, acts upon the digestive tract.

In particular, Orlistat^{®} reduces the absorption of dietary fat preventing the absorption and breakdown of triglycerides by inhibiting gastric lipases, namely the enzymes that break triglycerides down into simpler fragments more easily absorbed by the intestinal mucosa (fatty acids and monoglycerides).

Orlistat^{®} also has some important drawbacks.

A first important drawback lies in the fact that the undigested triglycerides lead to the problems typically associated with steatorrhea and, in some cases, flatulence, incontinence, excess fat in the faeces and faecal urgency.

Another problem consists of the fact that it tends to interfere with hypoglycaemic drugs and thus cannot be used by people suffering from diabetes.

In view of the above considerations, this drug (Orlistat^{®}) is currently the only drug marketed in Italy to treat excess of weight.

In this situation the technical purpose of the present invention is to develop a nutraceutical compound for treating excess of weight able to substantially overcome the inconveniences mentioned above.

Within the sphere of said technical purpose one important aim of the invention is to obtain a nutraceutical compound which substantially has no contraindications and can thus be used by practically everyone.

The technical purpose and specified aims are achieved with a nutraceutical compound for treating excess of weight as claimed in the appended Claim 1. Preferred embodiments are described in the dependent claims.

In particular, the invention concerns a nutraceutical compound or functional foodstuff or pharma food suitable for use to make pills, tablets, powders dissolvable in a solution such as, for example, water.

The nutraceutical compound according to the invention comprises a first active component, suitable to regulate the absorption and metabolism of carbohydrates and lipids, and a second active component suitable to control glycaemic metabolism.

The first active component comprises an ingredient that is widely available in nature, betulin, lup-20(30)-ene-3β, 28-diol, also known as betulinol or betulinic acid. This is a pentacyclic triterpene alcohol with a lupane skeleton, structurally similar to cholesterol. The characteristics of the lupane skeleton are the five-membered ring and the isopropylidene group.

Betulin is extracted on an industrial scale from the bark of the birch tree, with an extraction yield of up to 30% of dry weight, using high boiling hydrocarbon solvents, with water azeotrops of alcohols.

The best-known derivative of betulin is betulinic acid (AB) which can be extracted from numerous varieties of fruits and vegetables or derived directly from betulin and which has been reported to have anti-diabetic and slimming properties. Betulin extracts can contain a variable amount (3-5%) of betulinic acid.

The second active component is able to control glycaemic metabolism and, in particular, significantly reduce the transformation of sugars (polysaccharides) into glucose and, consequently, slow down the absorption of sugar into the bloodstream.

Said second active component comprises an active ingredient defined by 1-deoxynojirimycin (1-deoxynojirimycin or DNJ), a natural extract obtained from blackberries, the silkworm or the white mulberry.

In the nutraceutical compound, the ratio between the content of the first active component, betulin, and the content of the second active component, 1-deoxynojirimycin, is substantially comprised between 0.05 and 1.5, preferably between 0.3 and 0.8 and, yet more preferably, substantially equal to 0.5.

In particular, the first active component and the second active component can be taken at a daily dose substantially comprised between 50 mg/day and 500 mg/day and between 100 mg/day and 1000 mg/day and, preferably, substantially comprised between 150 mg/day and 350 mg/day and between 400 mg/day and 700 mg/day. In particular, given the complexity of obtaining purified extracts of the first and of the second active component, that is to have extracts composed substantially exclusively of said components, said doses will, in the case of a 20% extract, be comprised between 250 mg/day and 2500 mg/day, for the first active component, and between 500 mg/day and 5000 mg/day for the second active component.

In order to permit correct use, if, for example, the nutraceutical compound is in the form of pills suitable to be taken twice a day, and if the dosages of the first active component and of the second active component are respectively practically equal to 200 mg/day and 400 mg/day, each tablet/pill will have a nutraceutical compound in which the content of the first active component and of the second active component are, respectively, substantially equal to 100 mg/day and 200 mg/day which, in the case of a 20% extract, is equivalent, respectively, to a content of 500 mg/day and 1000 mg/day.

To demonstrate the efficacy of the nutraceutical compound comprising the two active components, namely betulin (B) and 1-deoxynojirimycin (DNJ), some experiments conducted by the applicant are reported below.

In detail, two experiments are described, which were conducted on CD-1 mice (as per the monograph in FRONTIERS IN NEUROENDOCRINOLOGY, IF2006.11.4) and on ob/ob mice (monogenic mice with a genetic defect in the ob gene) divided into cages. More in detail, each cage was maintained at an ambient temperature of 24±2°C at a 12-hour light/darkness cycle and contained four animals belonging to one of the two types and distributed so as to have substantially equivalent body weights in the two cages.

Each group of mice was treated with a particular diet, for six weeks. In particular, the CD-1 mice were divided into five groups that were treated, respectively, with:
- ND: a normal diet, i.e. not enriched with fat;
- HFD: a high-fat diet comprising: 15 g normal rodent pellets, 10 g roast hazelnuts, 10 g milk chocolate and 0.5 g corn biscuits;
- HFD+10 mg/L of B and DNJ 10 mg/L dissolved in drinking water (low dose);
- HFD+20 mg/L of B and DNJ 20 mg/L dissolved in drinking water (high dose); and
- HFD + 50 mg/L of Sibutramine^{®} dissolved in drinking water.

The ob/ob mice were divided into four groups treated with:
- ND: a normal diet plus water gavage;
- HFD and gavage with 10 mg/kg of B and 20 mg/kg of DNJ (low dose);
- HFD and gavage with 20 mg/kg of B and 40 mg/kg of DNJ (high dose);
- HFD and gavage with 20 mg/kg of Sibutramine^{®}.

At the end of the experimental period (6 weeks) the liver, abdominal fat (epididymal + parametrial) and whole blood from the retroocular region from which, in turn, plasma and serum were obtained, were removed and analysed. In particular, the results obtained with the CD-1 mice are reported in tables 1 and 2 below.

In particular, upon analysis, the CD-1 mice treated with the HFD+B-DNJ at the low dose, with the HFD+B-DNJ at the high dose and with Sibutramine^{®} were found to weigh, respectively, 1.4%, 32% and 13,6% less than those treated with the HFD alone, as shown in table 1. More in particular, from the comparison between the various HFD treatments and the ND treatment, abdominal fat, in the HFD+B-DNJ at the low dose and at the high dose was slightly greater than in the ND group, whereas in the case of HFD+Sibutramine^{®}, the abdominal fat decreased by just 40% with respect to the untreated HFD group.

**Table 1: analysis of biological variables in CD-1 mice**

| **CD**-**1 mice** | **ND** | **HFD** | **HFD low dose** | **HFD high dose** | **HFD sibutramine** |
|---|---|---|---|---|---|
| Initial weight [g] | 23.9±1.6 | 24.1±1.5 | 23.8±1.3 | 24.0±1.5 | 24.7±2.0 |
| Final weight [g] | 36.7±2.1 | 47.3±3.2° | 44.1±2.6*° | 39.8±2.2**° | 44.7±1.9* |
| food/week | 34.8±2.4 | 30.2±1.9° | 28.3±2.7* | 28.0±2.1** | 27.5±2.1** |
| water ml/week | 40.5±2.7 | 42.3±1.7 | 39.7±2.2 | 37.5±1.4 | 38.8+4.2 |
| Abdominal fat mg/10 g of weight | 240.5±40.3 | 767.3±100.5° | 421.5±96.3°* | 300.7±66.4* | 490.3 ±121.7 |
| Liver weight (mg/10 g of body weight) | 300.5±18.3 | 440.3±38.1° | 320.2±17.4* | 310.6±14.3** | 330.6±14.7* |
| ALTU/L | 40.6±11.7 | 42.4±16.3 | 40.0±11.7 | 41.7±12.5 | 42.3±15.1 |
| ASTU/L | 99.6±15.4 | 101.5±13.5 | 106.5±14.3 | 103.1±11.5 | 138.3±12.6* |
| Alkaline phosphatise | 82.5±13.7 | 94.3±12.2 | 94.3±12.2 | 90.1±11.4 | 110.3±16.4* |

| | | | | | |
|---|---|---|---|---|---|
| °p < 0.01 vs control group; *p < 0.01 vs HFD; **p < 0.01 vs sibutramine | | | | | |

Observing table 1, it also emerged that liver weight, which increased greatly with the HFD, remained close to the norm at both the high and low doses of B/DNJ and with Sibutramine^{®}.

A further result that emerged from said table is the absence of any particular changes in data for hepatic enzymes in any of the groups.

**Table 2:effects on plasma lipids, insulin, leptin and intestinal enzymes**

| **CD-1 mice** | **ND** | **HFD** | **HFD low dose** | **HFD high dose** | **HFD sibutramine** |
|---|---|---|---|---|---|
| Cholesterol mg/dl | 96.3±3.4 | 210.5±8.7° | 176.5±5.8°* | 148.3±8.4° ** | 183.6±45*° |
| TG mg/dl | 101.5±6.3 | 240.7±16.5° | 200.7±11.5°* | 150.3±13.5 °**⁺ | 193.4±13.5°* |
| Glycaemia mg/dl | 84.5±6.2 | 138.2±14.3° | 101.3±7.9°* | 94.1±8.4** | 115.7±18.2°* |
| Insulin pg/ml | 40.3±6.7 | 111.5±9.8° | 122.3±7.3° | 131.4±9.2° * | 126.8±11.5°* |
| Leptin ng/ml | 1.2±0.4 | 3.3±0.9* | 4.4±1.7°*⁺ | 4.7±2.1°*⁺ | 3.1±1.1* |
| Amylase U/L | 1452±282 | 1684±222° | 1396±188* | 1368±206 | 1406±248 |
| Lipase U/L | 256±49 | 288±71° | 264±38 | 251±44* | 247±37* |

| | | | | | |
|---|---|---|---|---|---|
| ° p < 0.01 vs control group; p* < 0.01 vs HFD; **p < 0.001 vs HFD; ⁺p < 0.001 vs sibutramine | | | | | |

From table 2 and, in detail, from the examination of the lipidic parameters, it also emerged that levels of cholesterol, triglycerides and glycaemia in the CD-1 mice fell more markedly with the two nutraceutical treatments (B-DNJ) and to a lesser extent with Sibutramine^{®}. Another finding that was observed was that insulinaemia, that is blood insulin levels, increased greatly with the HFD, whereas no particular changes were observed with the use of the nutraceutical compounds of AB-DNJ and a moderate reduction with Sibutramine^{®}.

Lastly, leptin levels increased dramatically with the HFD, and to a greater extent with the highest doses of AB + DNJ, but fell slightly with Sibutramine^{®} compared to the HFD group, although the result was not statistically significant with respect to mice treated with the ND.

After examining the CD-1 mice, the ob/ob mice were examined and the results are shown in tables 3 and 4.

As reported in table 3, the examination of the ob/ob mice revealed a very slight increase in weight in the HFD group treated with the nutraceutical compound at the high dose and, to a lesser extent, in the group treated with Sibutramine^{®}.

**Table 3: analysis of biological data in ob/ob mice**

| **Ob/ob mice** | **ND** | **HFD low dose** | **HFD high dose** | **HFD sibutramine** |
|---|---|---|---|---|
| Initial weight [g] | 40.7±3.3 | 39.9±2.9 | 40.2±3.7 | 40.8±3.5 |
| Final weight [g] | 49.3±5.6 | 47.8±3.6* | 45.2±3.1** | 46.5±2.6*° |
| Final net intake of food/week | 44.3±5.5 | 42.5±4.2 | 41.1±2.7*° | 40.8±5.1*° |
| Net intake of water ml/week | 40.1±4.9 | 41.3±3.6 | 42.4±4.0 | 41.7±4.8 |
| Epididymal fat mg/10 g of weight | 245±3.6 | 182±41** | 167±59** | 210±44 |
| Abdominal fat [g] | 7.32±1.96 | 6.88±1.36 | 5.44±0.32* | 6.38±1.96 |
| Liver weight (mg/10 g of body weight) | 982±84 | 999±106 | 1084±145 | 1053±201 |
| ALT U/L | 44.3±4.8 | 52.7±6.5 | 57.2±5.3 | 54.1±5.9 |
| AST | 62.5±7.1 | 66.2±5.9 | 64.3±4.3 | 67.1±7.1 |
| ALP | 103.4±8.7 | 105.4±9.8 | 104.3±6.7 | 109.5±8.7 |

| | | | | |
|---|---|---|---|---|
| °p < 0.01 vs control group; *p < 0.01 vs HFD; **p < 0.001 vs HFD | | | | |

From said analyses it emerged that in ob/ob mice treated with B/DNJ or Sibutramine^{®}, the intake of food/week fell by around 10% compared to those treated with the HFD, while both groups consumed the same amount of water, which means the reduction in body weight was largely a consequence of the reduction in epididymal fat.

**Table 4: Effect of B/DNJ on plasma lipids, glycaemia and insulin levels in ob/ob mice**

| **Ob/ob mice** | **ND** | **HFD low dose** | **HFD high dose** | **HFD sibutramine** |
|---|---|---|---|---|
| Total cholesterol mg/dl | 142.4+4.3 | 140.7±6.3 | 129.5±5.1* | 138.5±3.9 |
| TG mg/dl | 115.3±7.2 | 107.1±9.2 | 100.3±6.5* | 112.4±8.3 |
| Glycaemia mg/dl | 196.4±10.3 | 177.2±13.4* | 158.4±6.7**⁺ | 188.3±12.5 |
| Insulin ng/ml | 72.3±14.1 | 66.4±12.2 | 60.3±10.6*⁺ | 70.4±12.6 |

| | | | | |
|---|---|---|---|---|
| °p < 0.01 vs HFD and control group; *p < 0.001 vs HFD; **p < 0.01 vs sibutramine Moreover, from the analyses reported in table 4, it can be noted that in the ob/ob mice, unlike in the CD-1 mice, the daily gavage determined a clear reduction in glycaemic levels even compared to mice treated with the ND. | | | | |

The change in insulin levels which, especially in the case of the HFD with the high dose, even fell compared to those of mice treated with the ND, is of particular importance.

The invention achieves some important advantages.

As demonstrated by the results of the experiments reported above, the advantageous combination of the two elements significantly increases the advantages.

Particular improvements were observed in insulin sensitivity, weight loss, reduction of abdominal fat content with a marked effect on triglyceride levels.

In particular, the study and conception of a nutraceutical compound having the aforesaid two components have made it possible to obtain a compound with considerable potential for reducing body weight while having a limited effect on food intake, thus without interfering with the nervous system.

Furthermore, as emerged from the study on normal animals treated with a high-fat diet and on ob/ob mice, moderate falls in insulin levels were reported, while leptin levels rose in the CD-1 mice. This demonstrates that the particular combination of betulin (B) and 1-deoxynojirimycin (DNJ) can have a potential endocrine-metabolic effect of great significance in the treatment of obesity, probably by exploiting the antagonism between leptin and insulin in regulating food intake.

In conclusion, the association of said active components is of significant interest in clinical practice as a treatment to be associated with a low-calorie diet and any active components, such as high-fibre vegetables, proteins and pulses or other components that have proven to be active in treating obesity.

A further advantage lies in the particular ratio between the two components, that is between the betulin and the 1-deoxynojirimycin which, as emerged from the aforesaid study, permits the achievement of a particularly effective action.

In particular, the possibility of regulating the absorption and metabolism of carbohydrates and lipids, given by the betulin, and the possibility of controlling glycaemic metabolism, given by the 1-deoxynojirimycin, make it possible to intervene, in a particularly well-balanced manner, on practically all of the indices which determine an increase in body weight and which, thus, cause the problems described above.

A further advantage, which emerged clearly from the experiment, lies in the fact that thanks to the particular combination of betulin and 1-deoxynojirimycin and to their particular mixing ratio, the nutraceutical compound, does not cause any substantial nutritional imbalances (the food/water intake is not altered) and, thus, would not cause the patient to have an unbalanced diet.

The nutraceutical compound can thus easily be adapted according to any kind of diet without causing harmful side effects.

## Claims

1. A nutraceutical compound for use in treating excess of weight **characterised in that** it comprises a first component comprising a first active ingredient defined by betulin and suitable to regulate the absorption and metabolism of carbohydrates and lipids and a second comprising a second active ingredient defined by 1-deoxynojirimycin suitable to control glycaemic metabolism.

2. A nutraceutical compound according to claim 1, wherein said betulin is naturally extracted.

3. A nutraceutical compound as claimed in claim 1, comprising betulinic acid in an amount of less than or equal to 10%.

4. A nutraceutical compound as claimed in the preceding claim, wherein the betulin is extracted from the bark of the birch tree.

5. A nutraceutical compound as claimed in one or more of the preceding claims, wherein said 1-deoxynojirimycin is naturally extracted.

6. A nutraceutical compound as claimed in the preceding claim, wherein said 1-deoxynojirimycin is extracted from blackberries.

7. A nutraceutical compound as claimed in claim 5 wherein said 1-deoxynojirimycin is extracted from the silkworm.

8. A nutraceutical compound as claimed in one or more of the preceding claims, wherein the ratio between the content of terpenoids and said 1-deoxynojirimycin is comprised between 0.05 and 1.5.

9. A nutraceutical compound according to the preceding claim, wherein said ratio is comprised between 0.3 and 0.8.

10. A nutraceutical compound according to the preceding claim, wherein said ratio is substantially equal to 0.5.

## Patentansprüche

1. Nutrazeutikum zur Behandlung von Übergewicht, **dadurch gekennzeichnet, dass** es einen ersten Bestandteil mit einem durch Betulin definierten Wirkstoff umfasst, der geeignet ist, die Aufnahme und den Stoffwechsel von Kohlenhydraten und Fetten zu regeln, und einen zweiten Bestandteil mit einem zweiten, durch 1-deoxynojirimycin definierten Wirkstoff, einem aktiven Bestandteil, der geeignet ist, den Glukosestoffwechsel zu steuern.

2. Nutrazeutikum nach Anspruch 1, bei dem das genannte Betulin natürlich gewonnen wird.

3. Nutrazeutikum nach Anspruch 1, das Betulinsäure zu ca. 10 % oder in geringerer Menge umfasst.

4. Nutrazeutikum nach dem vorangegangenen Anspruch, bei dem das Betulin aus Birkenrinde gewonnen wird.

5. Nutrazeutikum nach einem oder mehreren der vorangegangenen Ansprüche, bei dem das genannte 1-deoxynojirimycin natürlich gewonnen wird.

6. Nutrazeutikum nach Anspruch 5, bei dem das genannte 1-deoxynojirimycin aus Brombeeren gewonnen wird.

7. Nutrazeutikum nach dem vorangegangenen Anspruch, bei dem das genannte 1-deoxynojirimycin aus der Seidenraupe gewonnen wird.

8. Nutrazeutikum nach einem oder mehreren der vorangegangenen Ansprüche, bei dem das Verhältnis zwischen dem Inhalt der genannten Terpenoide und dem genannten 1-deoxynojirimycin im Wesentlichen zwischen 0,05 und 1,5 liegt.

9. Nutrazeutikum nach dem vorangegangenen Anspruch, bei dem das genannte Verhältnis im Wesentlichen zwischen 0,3 und 0,8 liegt.

10. Nutrazeutikum nach dem vorangegangenen Anspruch, bei dem das genannte Verhältnis im Wesentlichen bei 0,5 liegt.

## Revendications

1. Alicament pour le traitement d'excès de poids **caractérisé en ce qu'**il comprend un premier composant comprenant un premier principe actif définit de bétuline et apte à régler l'absorption et le métabolisme de glucides et lipides et un deuxième composant comprenant un deuxième principe actif définit de 1-désoxynojirimicine apte à contrôler le métabolisme glycémique.

2. Alicament selon la revendication 1, dans lequel ladite bétuline est d'extraction naturelle.

3. Alicament selon la revendication 1, comprenant acide bétulinique en quantité inférieure ou environ égale à 10 %.

4. Alicament selon la revendication précédente, dans lequel la bétuline et extraite de l'écorce de bouleau.

5. Alicament selon une ou plusieurs des revendications précédentes dans lequel ladite 1-désoxynojirimicine est d'extraction naturelle.

6. Alicament selon la revendication 5, dans lequel ladite 1-désoxynojirimicine est extraite des mûres.

7. Alicament selon la revendication précédente, dans lequel ladite 1-désoxynojirimicine est extraite de ver à soie.

8. Alicament selon une ou plusieurs des revendications précédentes, dans lequel le rapport entre le contenu desdits terpénoïdes et de ladite 1-désoxynojirimicine est essentiellement compris entre 0,05 et 1,5.

9. Alicament selon la revendication précédente, dans lequel ledit rapport est essentiellement compris entre 0,3 et 0,8.

10. Alicament selon la revendication précédente, dans lequel ledit rapport est essentiellement égal à 0,5.
